# EUROPEAN PATENT APPLICATION

(11) **EP 1 481 986 A1**
(43) Date of publication of application: **01.12.2004**
(21) Application number: 03012422.6
(22) Date of filing: 30.05.2003
(51) Int. Cl.: C07K 14/195, C12P 1/06, A61K 38/02, A61P 31/04, A23K 1/17

(54) **Antibiotic 97518, pharmaceutically acceptable salts and compositions, and use thereof**

(71) Applicant: Vicuron Pharmaceuticals, Inc., King of Prussia, PA 19406 (US)
(72) Inventor: Losi, Daniele, 22069 Rovellasca (CO) (IT); Cavaletti, Linda, 22069 Rovellasca (CO) (IT); Lazzarini, Ameriga, 20020 Rescaldina (MI) (IT); Candiani, Gianpaolo, 20064 Gorgonzola (MI) (IT); Castiglione, Franca, 21047 Saronno (VA) (IT); Marinelli, Flavia, 20148 Milano (MI) (IT)
(74) Representative: Sgarbi, Renato

(57) **Abstract**

The invention relates to an antibiotic substance of microbial origin, arbitrarily denominated 97518 which is produced by fermentation of *Planomonospora* sp. DSM 14920, the pharmaceutically acceptable salts and compositions thereof, and their use as an antibacterial agent having inhibitory activity versus susceptible microbes or as animal growth promoter.

## Description

The present invention concerns an antibiotic substance of microbial origin, arbitrarily denominated antibiotic 97518, the pharmaceutically acceptable salts and compositions thereof, and their use as antibacterial agents.

Another object of the present invention is a process for preparing antibiotic 97518 which includes culturing *Planomonospora* sp. DSM 14920 or a variant or mutant thereof maintaining the genetic ability of producing said antibiotic, recovering the antibiotic of the invention from the mycelium and/or from the fermentation broth, and isolating pure substance by chromatographic means.

Antibiotic 97518 is a novel antimicrobial agent with a peptide structure containing lanthionine and methyl lanthionine as constituents. It was found to inhibit cell wall biosynthesis in bacteria. These are the typical characteristics of type B lantibiotic group.

Lantibiotics are peptides, which contain the thioether amino acid lanthionine as well as several other modified amino acids (H.G. Sahl and G.Bierbaum. Lantibiotics: biosynthesis and biological activities of uniquely modified peptides from gram-positive bacteria- Ann.Rev. Microbiol. 52 (1998)41-79). The majority of known lantibiotics have antibacterial activity, although some have been reported as active on different pharamcological targets. The antibacterial lantibiotics can be broadly divided into two groups on the basis of their structures: type-A lantibiotics are typically elongated, amphiphilic peptides, while type-B lantibiotics are compact and globular (O. McAuliffe, R.P. Ross and C. Hill. Lantibiotics: structure, biosynthesis and mode of action- FEMS Microb. Rev. 25(2001) 285-308). Nisin is the typical representative of type A lantibiotic, whereas actagardine (gardimycin) and mersacidin belong to the type B lantibiotic subclass. Both nisin-type and mersacidin-type lantibiotics interact with the membrane-bound peptidoglycan precursors lipid II, although the two classes differ in the effects they produce in the bacterial proliferation process. Nisin-type lantibiotics primarily kill bacteria by permeabilization of the cytoplasmic membrane (H. Brotz, M. Josten, I. Wiedemann, U. Schneider, F. Gotz, G. Bierbaum and H.G. Sahl. Role of lipid-bound peptidoglycan precursors in the formation of pores by nisin, epidermin and other lantibiotics Mol. Microbiol. 30 (1998) 317-27), whereas mersacidin-type lantibiotics primary kill the bacterial cell by inhibiting the cell wall biosynthesis (H. Brotz, G. Bierbaum, K. Leopold, P.E. Reynolds and H.G. Sahl. The lantibiotic mersacidin inhibits peptidoglycan synthesis by targeting lipid II. Antimicrob Agents Chemother. 42 (1998) 154-60).

### STRAIN AND FERMENTATION

*Planomonospora sp.* DSM 14920 was isolated in the environment and deposited on 2002/03/20, with the DSMZ, (Deutsche Sammlung von Mikroorganismen und Zellkulturen GmbH, Mascheroder Weg 1b, D-38124 Braunschweig, Germany), under the provision of the Budapest Treaty. The strain was accorded accession number DSM 14920.

The production of antibiotic 97518 is achieved by cultivating a *Planomonospora* strain maintaining the genetic ability of producing it, i.e. *Planomonospora* sp. DSM 14920 or a variant or mutant thereof; isolating the resulting antibiotic from the culture broth and/or from the fermentation broth; and purifying the isolated antibiotic by chromatographic means. In any case, it is preferred to produce antibiotic 97518 under aerobic conditions in an aqueous nutrient medium containing easy assimilated sources of carbon, nitrogen, and inorganic salts. Many of the nutrient media usually employed in the fermentation field can be used, however certain media are preferred.

Preferred carbon sources are sucrose, inositol, fructose, glucose, xylose, and the like. Preferred nitrogen sources are soybean meal, peptone, meat extract, yeast extract, tryptone, aminoacids, hydrolized casein and the like. Among the inorganic salts which can be incorporated in the culture media, there are the customary soluble salts capable of yielding sodium, potassium, iron, zinc, cobalt, magnesium, calcium, ammonium, chloride, carbonate, sulphate, phosphate, nitrate, and the like ions.

Preferably, the strain producing antibiotic 97518 is pre-cultured in a fermentation tube or in a shake flask, then the culture is used to inoculate jar fermentors for the production of substantial quantities of substances. The medium used for the pre-culture can be the same as that employed for larger fermentations, but other media can also be employed. The strain producing antibiotic 97518 can be grown at temperature between 22°C and 48°C, optimal temperatures being around 28°C. During the fermentation, antibiotic 97518 production can be monitored by bioassay on susceptible microorganisms and/or by HPLC analyses. Maximum production of 97518 generally occurs after circa 96 hours and before the 300 hours of fermentation. Antibiotic 97518 produced by cultivating *Planomonospora* sp. DSM 14920 or a variant or mutant thereof producing antibiotic 97518, can be isolated from the culture broths and/or the mycelium.

MORPHOLOGICAL CHARACTERISTICS OF *PLANOMONOSPORA* sp. DSM 14920 *Planomonospora sp.* DSM 14920 grows well on many standard solid media. Microscopic examination and cell dimensions were measured using the culture grown on Soil Extract Agar (N.S.Makkar and T. Cross. Actinoplanes in soil and on plant litter from freshwater habitats J. Appl. Bacteriol. 52 (1982) 209-218)

In liquid culture (V6 medium,composition in g/l:dextrose 22, meat extract 5, yeast extract 5, casein 3, NaCl 1.5) no fragmentation of the mycelium is observed after 6 days of growth at 28°C. Microscopic examination on Soil Extract Agar after 21 days of incubation at 28°C reveals a not fragmented vegetative mycelium and a scanty developed aerial mycelium with abundant presence of sporangial clusters. These resemble rows of bananas being made up by two parallel rows of rod-shaped sporangia disposed parallel to the longitudinal axis. Sporangial clusters are borne by sporangiophores, most of them emerging directly from the vegetative mycelium and are made by 10 to 30 sporangia (5 to 15 per row); single sporangia or little clusters (< 10 sporangia) are rarely observed. Sporangia are oblong (3.5 to 4.4 by 1.1 to 1.5 µm) and contain single, slightly curved, cylindrical spores (3 to 4 by 1 to 1.3 µm). Spores became motile after immersion in water for 20 to 90 minutes (the most after 1 hour); motility remains for many (up to 12) hours.

CULTURAL CHARACTERISTICS OF *Planomonospora* sp. DSM 14920 *Planomonospora* sp. DSM 14920 was grown for four days in AF/MS liquid medium (see Example 1 for medium composition), then stored at -20°C as stock culture. A 2 ml aliquot was thawed and used to inoculate 100 ml of V6 liquid medium. After 6 days of growth at 28°C and 200 rpm, the mycelium was harvested by centrifugation and washed three times by sterile saline solution and then diluted to provide a suitable inoculum. Aliquots of the suspension were streaked in a cross-hatched manner onto various media recommended by Shirling and Gottlieb (E.B. Shirling and D. Gottlieb. Method for Characterization of Streptomyces species. Int. J. Syst. Bacteriol. 16 (1966) 313-340) and several media recommended by Waksman (S.A. Waksman The Actinomycetes. The Williams and Wilkins Co., Baltimore. Vol 2 (1961) 328-334).

The ability to use a variety of carbohydrates as a carbon and energy source was determined in Chelate Mineral Medium [CMM; K₂HPO₄ 1,2 g/l, KH₂PO₄ 0.62 g/l, CaCl₂x6H₂O 50 mg/l, MgSO₄x7H₂O 200 mg/l, NaCl 100 mg/l, (NH₄)₂SO₄ 500 mg/l, EDTA-Na₂x2H₂O 15.8 mg/l, ZnSO₄x7H₂O 7 mg/l, MnSO₄x4H₂O 1.8 mg/l, FeSO₄x7H₂O 1.6 mg/l, NaMoO₄x2H₂O 0.47 mg/l, CuSO₄x5H₂O 0.47 mg/l, CoCl₂x6H₂0 0.52 mg/l, Vitamins solution (Thiamine hydrochloride 25 mg/l, calcium pantotenate 250 mg/l, nicotinic acid 250 mg/l, biotin 0.5 mg/l, riboflavin 1.25 g/l, cyanocobalamin 6.25 mg/l, paraminobenzoic acid 25 mg/l, folic acid 500 mg/l, pyridoxal hydrochloride 500 mg/l) 2 ml/l, agar 15 g/l] containing the carbon source at the final concentration of 2% (w/v). NaCl tolerance as well as ability to grow at different temperatures was determined onto ISP2 medium. All media were incubated at 28°C for three weeks; descriptions are referred to 21 days unless specified. Colour was assessed in natural daylight, using the Colour Atlas of Maerz and Paul (A. Maerz and M.R. Paul. A Dictionary of Colour, 2nd edition (1950). McGraw-Hill Book Co. Inc.,New York). Ability to reduce nitrates to nitrites was evaluated in Nitrate Broth (ISP8 medium) after overnight incubation at 28°C in aerobic condition using Bacto-Nitrite Test Strips (Difco Labs, Detroit, USA), following the protocol suggested by the manufacturer.

Growth, colonial appearance, substrate and aerial mycelium colour and pigment production for strain *Planomonospora* sp.DSM 14920 are recorded in Table I. Vegetative growth is present on all media used, differently from the aerial mycelium that is present only on some of them. Most of the macroscopically visible aerial mycelium is constituted by the abundant presence of sporangia. No characteristic pigmentation is shown on any medium used. Physiological characteristics of the strain are presented in Table II. Abundant production of aerial mycelium and sporangia on ISP2 is present at temperatures higher than 28°C, but it is absent at 22°C. The ability to use various carbohydrates for growth is shown in Table III. On the basal medium CMM without any carbon source addition, vegetative growth is nearly absent but relatively abundant aerial mycelium is observed. No aerial mycelium production is indeed detectable in CMM added with glucose.

**Table I:**

| growth characteristics of *Planomonospora* sp. DSM 14920. | | |
|---|---|---|
| **MEDIUM** | **GROWTH & MORPHOLOGY** | **REVERSE COLOUR CODE** |
| **ISP 2** Yeast extract-Malt extract agar | Good growth; good production of thin, cream/beige (9C2) aerial mycelium, entirely covering the vegetative mycelium. Abundant presence of sporangia. No soluble pigments produced. | 10 G 3 beige-yellowish |
| **ISP 3** Oatmeal agar | Good growth, widely spread from area of streaks; good production of thin, pinkish (2A1 to 2A2) aerial mycelium; margins of vegetative mycelium enlarged out of the aerial one. Abundant presence of sporangia. No soluble pigments produced. | 10 A 1 whitish |
| **ISP 4** Inorganic salts- Starch agar | Good growth; abundant production of thin, cream/pinkish (2B1) aerial mycelium, entirely covering the vegetative mycelium. Abundant presence of sporangia. No soluble pigments produced. Starch hydrolysed. | 10 B 2 beige |
| **ISP 5** Glycerol-Asparagine agar | Discrete growth, thin, with feathered margins, yellowish/brown. No aerial mycelium produced. No soluble pigments produced. | 10 B 4 beige-pinkish |
| **ISP 6** Peptone-yeast extract-iron agar | Good growth, convolute, yellowish/brown. No aerial mycelium produced. Slight darkening of the medium. | 11 K 5 yellowish-brown |
| **ISP 7** Tyrosine agar | Discrete growth with feathered margins; discrete production of thin, beige, aerial mycelium. Slight production of brownish soluble pigment. | 11 H 5 yellowish-brown |
| **SYE** Starch-yeast extract agar | Scant growth, yellowish. No aerial mycelium produced. | 11 G 2 yellowish-brown |
| **CZ-GLU** Czapeck-glucose agar | Very scant growth, colourless. No aerial mycelium produced. | nd |
| **GAUZE 1** agar | Abundant growth, spreaded from area of streaks, with smooth surface; lemon, discolored at margins of streaks. Scant presence of white aerial mycelium close to the cross-hatched areas. No soluble pigments produced. | 10 E 2 beige-yellowish |
| **GAUZE 2** agar | Abundant growth, spreaded from area of streaks, wrinkled near the edges of streaks; brownish. No aerial mycelium produced. No soluble pigments produced. | 11 J 6 brownish-orange |
| **NA** Nutrient agar | Good growth, cream/yellowish, with wide, feathered margins. Scant presence of whitish aerial mycelium close to the ends of streaks. No soluble pigments produced. | 10 C 2 beige |
| **SM** Skimmed Milk agar | Abundant growth, slightly convolute; yellowish/brown. No aerial mycelium produced. No soluble pigments produced. Digestion of casein (halo of digestion diffused of - 3mm). | 10 H 4 beige-yellowish |
| **CaMal** Calcium Malate agar | Vegetative mycelium not visible. Discrete presence of a thin, white aerial mycelium. Abundant presence of sporangia. No calcium-malate hydrolysis. | nd |

**Table II:**

| physiological characteristics of *Planomonospora* sp. DSM 14920. | |
|---|---|
| **TEST** | **REACTION** |
| Starch hydrolysis | Positive |
| Casein hydrolysis | Positive |
| Calcium malate digestion | Positive |
| Litmus milk peptonization | Negative |
| Litmus milk coagulation | Negative |
| Gelatin liquefaction | Positive (10 days) |
| Tyrosine reaction | Positive (weak) |
| H₂S production (4 days): on ISP6 + lead acetate strips | Negative Positive |
| Nitrate reduction | Positive |
| NaCl % tolerance | ≤ 4 |
| Temperature range of growth | 22°C ≤ T ≤ 48°C Abundant presence of aerial mycelium at ≥ 28°C, absent at 22°C |

**Table III:**

| utilization of carbon sources by *Planomonospora* sp. DSM 14920. | | |
|---|---|---|
| **Carbon source** | **Vegetative Mycelium Growth** | **Aerial Mycelium Growth** |
| Arabinose | + | - |
| Fructose | ++ | +/- |
| Inositol | +++ | ++ |
| Mannitol | + | +/- |
| Raffinose | + | - |
| Rhamnose | + | + |
| Sucrose | +++ | ++ |
| Xylose | + | - |
| Glucose | + | - |
| No sugar | - | ++ |
| +++ abundant; ++ good growth; + moderate growth; - scant/no growth; AM: aerial mycelium production; ++ abundant; + good; +/- scant; - absent. | | |

CHEMOTAXONOMICAL CHARACTERISTICS OF *Planomonospora* sp. DSM 14920.

*Planomonospora* sp. DSM 14920 was grown in GYM medium (glucose 4g/l; yeast extract 4 g/l; malt extract 10 g/l) for four days at 28°C on a rotary shaker and the mycelium harvested, washed twice with sterile distilled water and subsequently freeze-dried. Analyses of amino acids and sugars were carried out according to the method of Staneck and Roberts, (J.L. Staneck and G.D. Roberts. Simplified approach to identification of aerobic actinomycetes by thin-layer chromatography. Appl. Microbiol. 28 (1974) 226-231). Menaquinones and polar lipids were extracted following the procedure of Minnikin et al. (D.E. Minnikin, A.G. O'Donnell, M. Goodfellow., G. Alderson, M. Athalye, A. Schaal and J.H. Parlett. An integrated procedure of isoprenoid quinones and polar lipids- J. Microbiol. Meth. 2 (1984) 233-241). Polar lipids were analysed by thin layer chromatography (D.E. Minnikin, V.Patel, L.Alshamaony, and M. Goodfellow. Polar lipid composition in the classification of *Nocardia* and related bacteria - Int. J. Syst. Bacteriol. 27 (1977) 104-117), menaquinones by HPLC (R.M. Kroppenstedt. Separation of bacterial menaquinones by HPLC using reverse phase RP18 and a silver loaded ion exchanger as stationary phase. J. Liquid. Chromat. 5 (1982) 2359-2367; R.M. Kroppenstedt. Fatty acid and menaquinone analysis of actinomycetes and related organisms. In: Chemical Methods in Bacterial Systematics, No 20 SAB Technical Series, pp.173-199, M. Goodfellow and D.E. Minnikin eds (1985), Academic Press, London) and fatty acid methyl esters by gas-liquid chromatography (L.T. Miller. A single derivatization method for bacterial fatty acid methyl esters including hydroxy acids J. Clin. Microbiol. 16 (1982) 584-586; M.Sasser. Identification of bacteria by gas chromatography of cellular fatty acids. USFCC News Letters 20 (1990) 1-6), respectively. The presence of mycolic acids was checked by the method of Minnikin et al.(D.E. Minnikin, L.Alshamaony, and M. Goodfellow. Differentiation of *Mycobacterium, Nocardia* and related taxa by thin layer chromatographic analysis of whole organism methanolyzates J. Gen. Microbiol. 88 (1975) 200-204). Whole cell hydrolyzates of strain DSM 14920 contain *meso*-diaminopimelic acid as the diammino acid of the peptidoglycan and the sugars galactose, ribose and traces of madurose. A Menaquinone with a di- hydrogenated isoprenoid chain of nine isoprene units [MK-9(H₂)] is found as the dominant isoprenoid quinone, significant amount of MK-9 and small amount of MK-9(H₄) is found in addition. The polar lipid pattern is composed of phosphatidylglycerol, diphosphatidylglycerol, phosphatidylinositol, phosphatidylethanolamine, hydroxyphosphatidylethanolamine and the n-acetylglucosamine-containing phosphoglycolipid (GluNu),i.e. phospholipid type IV according to Lechevalier et al.(H.A. Lechevalier, C. De Briève and M.P. Lechevalier. Chemotaxonomy of aerobic actinomycetes: phospholipid composition Biochem. Syst. Ecol. 5 (1977) 246-260). The fatty acid pattern is composed of iso/anteiso-branched fatty acids. Of diagnostic value is the combination of 10-methyl-branched C_{17:0} fatty acid together with 2-hydroxy-branched fatty acids i.e. fatty acid pattern 3c according to Kroppenstedt (R.M. Kroppenstedt. The genus *Nocardiopsis* In: The Prokariotes, Vol II pp.1139-1156 A. Balows, H. Truper, M. Dworkin, W. Harder and K.H. Schleifer eds (1992); New York, Springer-Verlag. Mycolic acids are not detected.

### IDENTITY OF STRAIN DSM 14920.

The strain producing antibiotic 97518 is assigned to the genus *Planomonospora,* family *Streptosporangiaceae* because of the following chemotaxonomical and morphological characteristics:
- Presence of meso-diaminopimelic acid in the cell wall and of galactose, ribose and traces of madurose in the whole cell hydrolizate, i.e Chemotype IIIB according to Lechevalier and Lechevalier (H.A. Lechevalier and M.P. Lechevalier. A critical evaluation of the genera of aerobic actinomycetes In: The Actinomycetales. pp. 393-405, H. Prausers ed. (1970), Jena, Gustav Fischer Verlag);
- phospholipid type IV according to Lechevalier et al.(H.A. Lechevalier, C. De Briève and M.P. Lechevalier. Chemotaxonomy of aerobic actinomycetes: phospholipid composition. Biochem. Syst. Ecol. 5 (1977) 246-260).
- fatty acid profile of 3c *sensu* Kroppenstedt (R.M. Kroppenstedt. The genus *Nocardiopsis* In: The Prokariotes, Vol II, pp.1139-1156. A. Balows, H. Truper, M. Dworkin, W. Harder and K.H. Schleifer eds (1992); New York, Springer-Verlag)
- absence of mycolic acids
- formation of typical *Planomonospora* sporangia.

As with other microorganisms, the characteristics of strain producing antibiotic 97518 are subject to variation. For example, artificial variants and mutants of the strain can be obtained by treatment with various known mutagens, such as U.V. rays, and chemicals such as nitrous acid, N-methyl-N'-nitro-N-nitrosoguanidine, and many others. All natural and artificial variants and mutants of strain *Planomonospora* sp. DSM 14920 capable of producing antibiotic 97518 are deemed equivalent to it for the purpose of this invention and therefore within the scope of invention.

### EXTRACTION AND PURIFICATION OF ANTIBIOTIC 97518

As mentioned above, antibiotic 97518 is found both in the mycelium and in the filtered fraction of the fermentation broth. The mycelium can be extracted with methanol and then filtered off. The methanol extract contains about 10% of the total amount of the antibiotic 97518.

The recovery of the compound from fermentation broth of the producing microorganism is conducted according to known *per se* techniques which include extraction with solvents, precipitation by adding non-solvents or by changing the pH of the solution, partition chromatography, reverse phase partition chromatography, ion-exchange chromatography, molecular exclusion chromatography and the like. A procedure for recovering the compounds of the invention from the fermentation broths includes extraction of 97518 antibiotic with water-immiscible organic solvents, followed by precipitation from the concentrated extracts, possibly by adding a precipitating agent.

The term "water-immiscible solvent" as used in this application, is intended to have the meaning currently given in the art to this and refers to solvents that, at the conditions of use, are slightly miscible or practically immiscible with water in a reasonably wide concentration range, suitable for the intended use.

Examples of water-immiscible organic solvents that can be used in the extraction of the compounds of the invention from the fermentation broths are:
alkanols of at least four carbon atoms which may be linear, branched or cyclic such as n-butanol, 1-pentanol, 2-pentanol, 3-pentanol, 1-hexanol, 2-hexanol, 3-hexanol, 3,3-dimethyl-1-butanol, 4-methyl-1-pentanol, 3-methyl-1-pentanol, 2,2-dimethyl-3-pentanol, 2,4-dimethyl-3-pentanol, 4,4-dimethyl-2-pentanol, 5-methyl-2-hexanol, 1-heptanol, 2-heptanol, 5-methyl-1-hexanol, 2-ethyl-1-hexanol, 2-methyl-3-hexanol, 1-octanol, 2-octanol, cyclopentanol, 2-cyclopentylethanol, 3-cyclopenthyl-1-propanol, cyclohexanol, cycloheptanol, cyclooctanol, 2,3-dimethyl-cyclohexanol,4-ethylcyclohexanol, cyclooctylmethanol, 6-methyl-5-hepten-2-ol, 1-nonanol, 2-nonanol, 1-decanol, 2-decanol, and 3-decanol; ketones of at least five carbon atoms such as methylisopropylketone, methylisobutylketone, methyl-n-amylketone, methylisoamylketone and mixtures thereof.

As known in the art, product extraction may be improved by adjusting the pH at an appropriate value, and/or salting and/or by adding a proper organic salt forming an ion pair with the antibiotic, which is soluble in the extraction solvent.

As known in the art, phase separation may be improved by salting the aqueous phase.

When, following an extraction, an organic phase is recovered containing a substantial amount of water, it may be convenient to azeotropically distill water from it. Generally, this requires adding a solvent capable of forming minimum azeotropic mixtures with water, followed by the addition of a precipitating agent to precipitate the desired product, if necessary. Representative examples of organic solvents capable of forming minimum azeotropic mixtures with water are: n-butanol, benzene, toluene, butyl ether, carbon tetrachloride, chloroform, cyclohexane, 2,5-dimethylfuran, hexane, and m-xylene; the preferred solvent being n-butanol.

Examples of precipitating agents from organic solutions containing antibiotic 97518 are petroleum ether, lower alkyl ethers, such as ethyl ether, propyl ether, and butyl ether, and lower alkyl ketones such as acetone.

According to a preferred procedure for recovering compound 97518 from the filtered fermentation broth, the aqueous solution can be contacted with an adsorption matrix followed by elution with a polar, water miscible solvent or a mixture thereof, removal of water by concentration to an oily residue under reduced pressure, and precipitation with a precipitating agent of the type already mentioned above.

Examples of adsorption matrixes that can be conveniently used in the recovery of the compounds of the invention, are polystyrene or mixed polystyrene-divinylbenzene resins (e.g. M112 or S112, Dow Chemical Co.; Amberlite XAD2 or XAD4, Rohm & Haas; Diaion HP 20, Mitsubishi), acrylic resins (e.g. XAD7 or XAD8, Rohm & Haas), polyamides such as polycaprolactames, nylons and cross-linked polyvinylpyrrolidones (e.g. Polyamide-CC 6, Polyamide-SC 6, Polyamide-CC 6.6, Polyamide-CC 6AC and Polyamide-SC 6AC, Macherey-Nagel & Co., Germany; PA 400, M.Woelm AG, Germany); and the polyvinylpirrolidone resin PVP-CL, (Aldrich Chemie GmbH & Co., KG, Germany) and controlled pore cross-linked dextrans (e.g. Sephadex LH-20, Pharmacia Fine Chemicals, AB). Preferably, polystyrene resins are employed, particularly preferred being the Diaion HP 20 resin.

In the case of use of polystyrene resins, polystyrene-divinylbenzene resins, or acrylic resins a preferred eluent is a water miscible solvent or its aqueous mixtures. The aqueous mixtures can contain buffers at appropriate pH value.

The term "water-miscible solvent" as used in this application, is intended to have the meaning currently given in the art of this term and refers to solvents that, at the conditions of use, are miscible with water in a reasonably wide concentration range. Examples of water-miscible organic solvents that can be used in the elution of the compounds of the invention are: lower alkanols, e.g. (C₁-C₃) alkanols such as methanol, ethanol, and propanol; phenyl (C₁-C₃) alkanols such as benzyl alcohol; lower ketones, e.g. (C₃-C₄) ketones such as acetone and ethyl methyl ketone; cyclic ethers such as dioxane and tetrahydrofuran; glycols and their products of partial etherification such as ethylene glycol, propylene glycol, and ethylene glycol monomethyl ether, lower amides such as dimethylformamide and diethylformamide; acetic acid, dimethylsulfoxide and acetonitrile.

Purification of the crude 97518 antibiotic, can be accomplished by any of the known per se techniques but is preferably conducted by means of chromatographic procedures.

Examples of these chromatographic procedures are those reported in relation to the recovery step and include also chromatography on stationary phases such as polyamide-type resins or silica gel, alumina, activated magnesium silicate an the like or reverse phase chromathography on silanized silica gel having various functional derivatizations, and eluting with water immiscible solvents or aqueous mixture of water miscible solvents of the kind mentioned above.

For instance, preparative HPLC chromatography may be employed, using RP-18 as stationary phase and a mixture of buffer HCOONH₄(20 mM) : CH₃CN as eluting system.

The active fractions recovered from the purification step are pooled together, concentrated under vacuum and precipitated by addition of a precipitating agent of the kind mentioned above.

As usual in this art, the production as well as the recovery and purification steps may be monitored by a variety of analytical procedures including inhibitory assay against susceptible microorganisms and analytical control using the HPLC.

A preferred analytical HPLC technique is performed on a Shimadzu instrument equipped with a Ultrasphere 250 x 4.6 mm column (Beckman Instruments Inc. Fullerton CA; USA) 5 micron particle size, eluted at 1 ml/min flow rate with mixtures of phase A (20 mM ammonium formate pH 4.5 buffer: CH₃CN 95:5) and phase B (20 mM ammonium formate pH 4.5 buffer: CH₃CN 5:95). The separation is performed by eluting with a linear gradient from 10% to 90% of phase B in 28 min. Detection was at 230 nm. Antibiotic 97518 typically eluted after 23.7 min.

Since compound 97518 contains acid and basic functions, it is capable of forming salts with suitable bases or acids according to conventional procedures and it may exist also in the form of inner salt. The antibiotic, when obtained in the acid form or in the form of inner salt, may be converted into a corresponding non-toxic pharmaceutically acceptable salt with bases. Suitable salts include the alkali and alkaline earth metal salts, typically the sodium, potassium, calcium and magnesium salts, and the ammonium and substituted ammonium salts. Representative substituted ammonium salts include primary, secondary or tertiary (C₁-C₄) alkylammonium and hydroxy (C₁-C₄) alkylammonium salts and, according to an embodiment of the present invention, the benzathine, procaine, hydrabamine and similar water insoluble, non-toxic, pharmaceutically acceptable salts. Another preferred class of salts of the compound of the present invention is represented by the basic addition salts with basic amino acids such as arginine or lysine, or aminosugars such as glucosamine and the like.

The alkali and alkaline earth metal salts are prepared according to the usual procedures commonly employed for preparing metal salts. As an example, antibiotic 97518, in the acid form or in the inner salt form, is dissolved into the minimum amount of a suitable solvent, typically a lower alkanol, or a lower alkanol water mixture, the stoichiometric amount of a suitable selected base is gradually added to the obtained solution and the obtained salt is precipitated by the addition of a non-solvent. The alkali or alkaline earth metal salt, which forms is then recovered by filtration or evaporation of the solvents.

Alternatively, these salts can be prepared in a substantially anhydrous form through lyophilization; in this case aqueous solutions containing the desired salts, resulting from the salification of antibiotic 97518 with a suitably selected alkali or alkaline earth metal carbonate or hydroxide in such a quantity as to obtain a pH comprised between 7.0 and 8.5 are filtered from any insolubles and lyophilized.

The organic ammonium salts can be prepared substantially following the above procedure by adding the properly selected amine to a solution of antibiotic 97518 in a suitable solvent and then evaporating off the solvent and the excess of the amine reagent or by lyophilizing the concentrate solution.

The addition salts of antibiotic 97518 with acids can be also prepared. Representative and suitable acid addition salts of the compounds of the invention include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, phthalic, tartaric, lauric, stearic, salicylic, methanesulfonic, benzenesulfonic, sorbic, picric, benzoic, cinnamic and the like acids. The addition salts of antibiotic 97518 with acids can be prepared in a substantially analogues manner as that employed for the preparation of the salts with bases but using the appropriately selected acid as reagent in the place of the base.

The pharmaceutically acceptable salts so formed are also part of this invention. "Pharmaceutically acceptable" salts are useful in the therapy of warm-blooded animals.

The transformation of the non salts (or internal salts) compound of the invention into the corresponding addition non-toxic salts, and the reverse, i.e. transformation of an addition salt of a compound of the invention into the non-salt form are within the ordinary technical skill and are encompassed by the present invention.

The compounds of the invention can be administered as such or in admixtures with pharmaceutically acceptable carriers and can also be administered in conjunction with other antimicrobial agents such as penicillins, cephalosporins, aminoglycosides and glycopeptides.

Conjunctive therapy, thus includes sequential, simultaneous and separate administration of the active compound in a way that the therapeutic effects of the first administered one is not entirely disappeared when the subsequent is administered.

The compounds of the invention, or its pharmaceutically acceptable addition salts, can be formulated into forms suitable for parenteral, oral or topical administration. For iv administration in the treatment of any infection involving a microorganism susceptible to the antibiotic, a parenteral formulation is, for instance, in water with an appropriate solubilising agent such as polypropylene glycol or dimethylacetamide and a surface-active agent such as polyoxyethylene sorbitan mono-oleate or polyethoxylated castor oil or cyclodextrins in sterile water for injection.

The antibiotic may also be used in a suitable pharmaceutical form such as a capsule, a tablet or an aqueous suspension for oral administration or with conventional creams or jellies for topical applications. The dosage of the active ingredient depends on many factors, which include type, age and conditions of the patient, specific active ingredient and formulation selected for the administration, administration schedule, etc. In general, effective antimicrobial dosages are employed per single unit dosage form.

Repeated applications/administrations, e.q. from 2 to 6 times a day, are in general preferred. An effective dosage may be in general in the range 1 - 30 mg/kg body weight/day.

Anyway, the prescribing physician will be able to determine the optimal dosage for a given patient in a given situation.

Besides the use as medicament in human and veterinary therapy, the compound of the invention can also be used as animal growth promoter. For this purpose, the compound of the invention and/or a non-toxic salt thereof is administered orally in a suitable feed. The exact concentration of the active agent employed is the one which is required to provide a growth promotant effective amount of active ingredient when normal amounts of feed are consumed.

The addition of the active compound of the invention to animal feed is preferably accomplished by preparing an appropriate feed premix containing the active compound in an effective amount and incorporating the premix into the complete ration. Alternatively, an intermediate concentrate or feed supplement containing the active ingredient can be blended into the feed.

The way in which such feed premixes and complete rations can be prepared and administered are described in reference books (such as "Applied Animal Nutrition", W.H. Freedman and CO., S. Francisco, U.S.A., 1969 or "Livestock Feeds and Feeding" 0 and B books, Corvallis, Ore., U.S.A., 1977).

### PHYSICO-CHEMICAL CHARACTERISTICS OF ANTIBIOTIC 97518

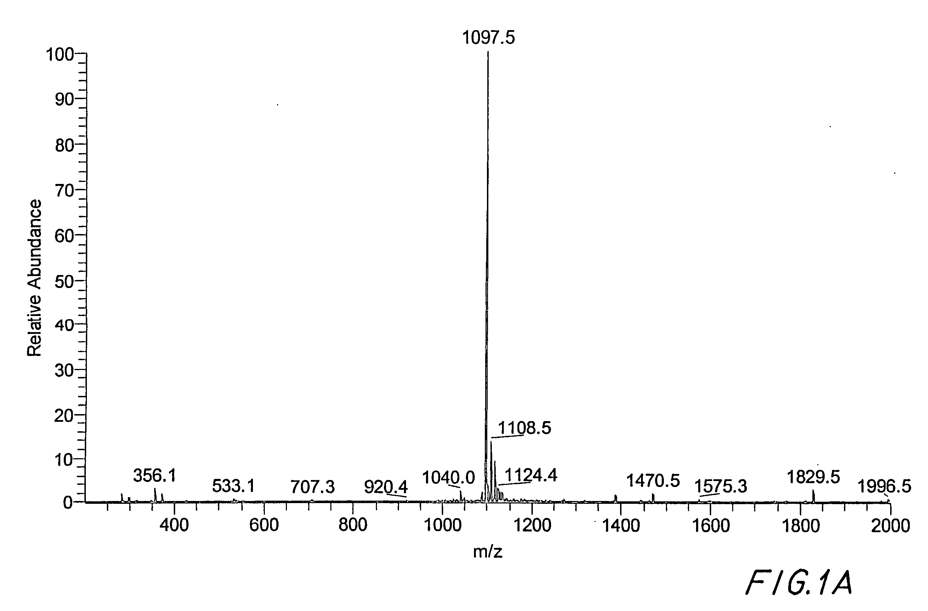

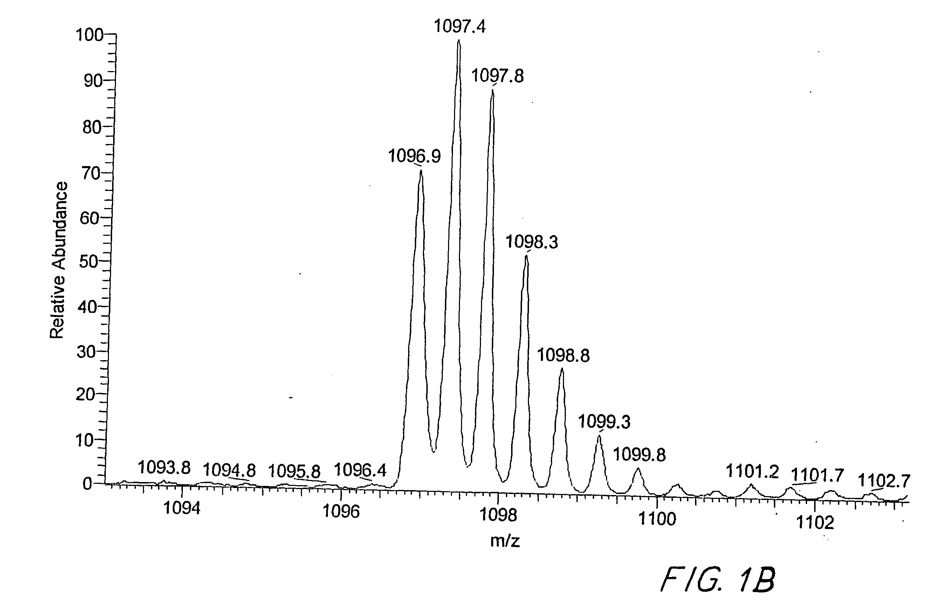

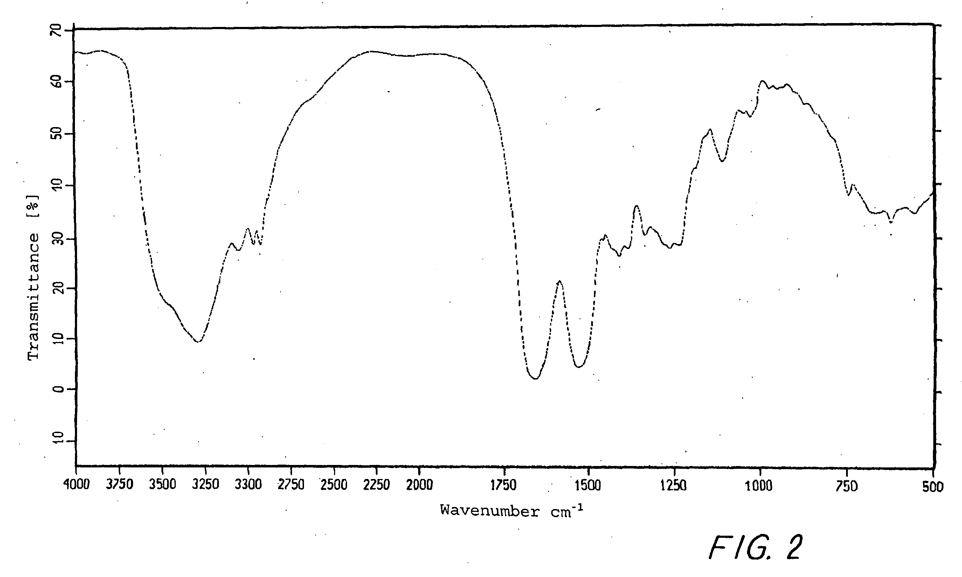

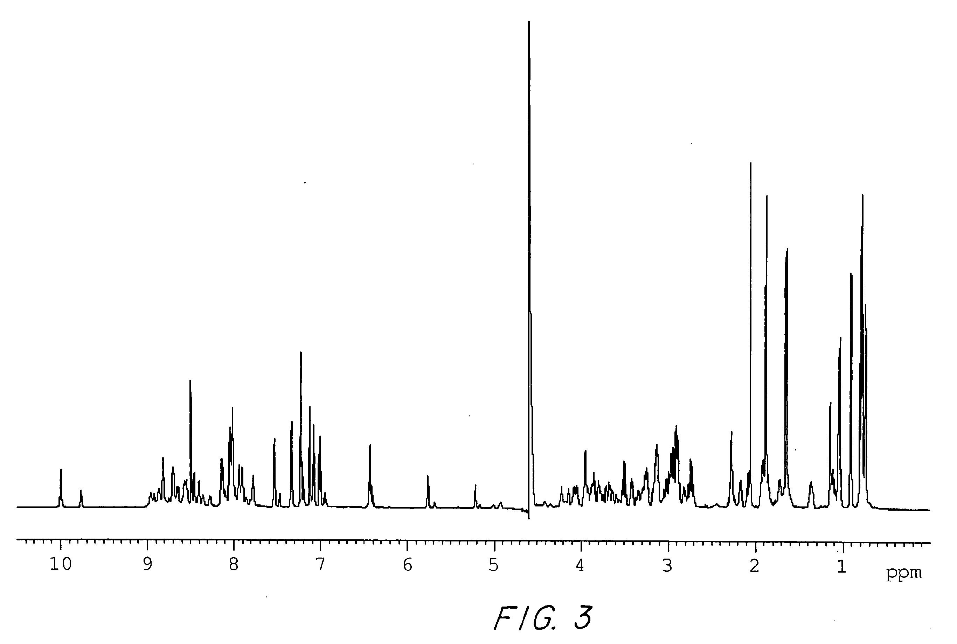

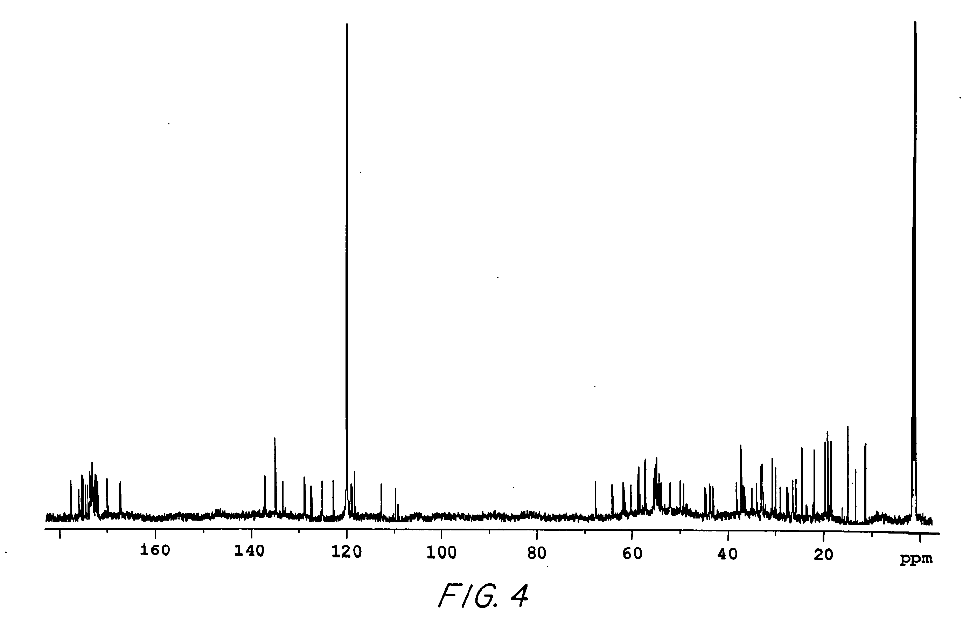

A) Mass spectrometry
   Antibiotic 97518 gives a doubly protonated ion at m/z=1097 in MS experiments on a Thermofinnigan LCQ deca instrument fitted with an electrospray source, using Thermofinnigan calibration mix. The electrospray conditions were: Spray Voltage: 4.5 kV; Capillary temperature: 200°C; Capillary Voltage: 28V; Infusion mode 5µl/min. Spectra were recorded from a 0.01mg/ml solution in acetonitrile water 50/50 (v/v).
   Antibiotic 97518 gives exact mass of the doubly protonated ion at m/z = 1096.8795 in experiments on a Bruker Daltonics APEX II, 4.7 Tesla spectrometer fitted with an electrospray source. FTMS conditions were: Electrospray: Analytica Source, Off-Axis Spray, 60 µl/h; Drying Gas 200° C; Capillary voltage: 70 V; Skimmer Voltage: 10 V; Accumulation: 40 Scans; Broad Band Mode: Resolution 20'000.
   Mass spectra are reported in Fig.1A and 1B.
B) Infrared spectrum, recorded in KBr with a Bruker FT-IR spectophotometer model IFS 48, exhibits the following absorption maxima (cm⁻¹): 3295; 3058; 2970; 2930; 1660; 1531; 1411; 1336; 1263; 1109. Infrared spectrum is reported in Fig.2
C) The U.V. spectrum, performed in MeOH with a Perkin-Elmer spectrophotometer Lambda 16, exhibits two shoulders at 279 and 288 nm.
D) ¹H-NMR spectrum was recorded in the mixture H₂O/D₂O/CD₃CN 80/10/10 (pH 2.6 HCl) at 25°C on a Bruker AMX 600 spectrometer applying a water suppression sequence. As internal standard the residual signal of acetonitrile at 1.99 ppm was considered.
   ¹H NMR spectrum of antibiotic 97518 exhibits the following groups of signals [δ=ppm multiplicity; (attribution)]: 0.79 d (CH₃), 0.84 d (CH₃), 0.86 d (CH₃), 0.97 d (CH₃), 1.1 d (CH₃), 1.20 d (CH₃), 1.71 d (CH₃), 2.33 q (CH₂), 2.4-3.6 m (peptidic alpha CH's), 3.6-5.1 m (peptidic alpha CH's), 5.27-5.82 s (CH₂), 6.49 q (CH), 6.9-8.57 t and d (aromatic CH's), 7.74-9.1 d and m (aromatic CH's and peptidic NH's). The ¹H-NMR spectrum of 97518 is reported in Fig.3.
E) ¹³C-NMR spectrum was recorded in the mixture H₂O/D₂O/CD₃CN 80/10/10 (pH 2.6 HCl) at 25°C on a Bruker AMX 600 spectrometer using as internal standard the residual signal of acetonitrile at 1.39, 118.69 ppm.
   ¹³C NMR spectrum of antibiotic 97518 exhibits the following groups [δ=ppm; (attribution)]: 10.27 - 20.95 (aliphatic CH₃ 's), 23.42 - 48.15 (aliphatic CH₂ 's), 48.77 - 66.6 (peptidic alpha CH's), 108 - 136 (aromatic and double bonds CH's and quaternary carbons), 166.12 - 176.3 (peptidic carbonyls).
   The ¹³C-NMR spectrum bb decoupled of 97518 is reported in Fig.4.

### AMINO ACIDS COMPOSITION OF ANTIBIOTIC 97518

Antibiotic 97518 was submitted to complete acidic hydrolysis and the aminoacids components of the antibiotic resistant to the acid treatment were identified. Acid labile amino acids are not detectable with this approach.

The hydrolysate (HCl 6N at 105°C for 24h) after derivatization with 6-aminoquinolyl-N-hydroxysuccinimidyl carbamate (AccQ-Tag™ Fluor reagent kit), was studied by HPLC-MS analysis in comparison with a mixture of standard amino acids similarly derivatized. The qualitative HPLC analysis was carried out on a liquid chromatography system with simultaneous DAD and MS detection.

The HPLC method had the following conditions:
Column:AccQ-Tag™ (Waters C18 NovoPak 4µm3.9 x 150mm)
Column temperature: 37°C
Flow: 1 mL/min.
Phase A:Ammonium acetate 140mM pH 5 (acetic acid)
Phase B:Water/acetonitrile 60/40 v/v
Elution program:

| Time (min.) | 0 | 5 | 30 | 35 | 40 | 41 |
|---|---|---|---|---|---|---|
| %B | 5 | 5 | 80 | 95 | 95 | 5 |

UV detection: 254nm MS conditions were the following:
Spectrometer: Finnigan LCQ Deca equipped with standard electrospray source.
Capillary temperature: 250°C
Source voltage: 4.70 KV
Source current: 80µA
Capillary voltage: -15V
In the LC/MS chromatograms obtained on the hydrolysate of antibiotic 97518, the following "acid-resistant" amino acids were identified:
lanthionin, methyllanthionin, glycine, threonine, proline, valine, glutamic acid, histidine, isoleucine.

### IN VITRO BIOLOGICAL ACTIVITY OF ANTIBIOTIC 97518

Antimicrobial activity of antibiotic 97518 was determined by using microbroth dilution method with standard U-bottom 96-well plates according to The National Committee for Clinical Laboratory Standards (Methods for Dilution Antimicrobial Susceptibility Tests for Bacteria that Grow Aerobically - Fifth Edition; Approved Standard. NCCLS document M7-A5 Vol.20 No. 2). Results are reported in Table VI. The strains used were clinical isolates or strains from American Type Culture Collection (ATCC). Powder of antibiotic 97518 was dissolved in DMSO; this solution was further diluted with distilled water. The media used were cation-adjusted Mueller Hinton broth (CAMHB) for *Staphylococcus aureus, Moraxella catarrhalis, Enterococci;* Todd Hewitt broth (THB) for *Streptococcus* *pyogenes, Streptococcus pneumoniae.* Inocula were 10⁴ CFU/ml. All strains were incubated at 35°C in air. Incubation time was 18-24 hours. After the incubation time visual reading was performed and the MIC was defined as the lower concentration that completely inhibited growth of the tested microorganism.

**TABLE VI:**

| **Microbiological activity of antibiotic 97518** | | |
|---|---|---|
| **MIC (mg/ml)** | | |
| STRAIN | **97518** | actagardine |
| 819 *Staphylococcus aureus* Smith ATCC 19636 | **8** | **64** |
| 100 *Staphylococcus aureus* ATCC 6538 209P | **2** | **32** |
| 1400 *Staphylococcus aureus* met R | **16** | **16** |
| 453 *Staphylococcus aureus* met R | **8** | **32** |
| 49 *Streptococcus pyogenes* cl.isol | **0.5** | **2** |
| 1190 *Streptococcus pneumoniae* cl.isol. | **2** | **16** |
| 1652 *Enterococcus faecium* cl.isol. Van A | **64** | **64** |
| 997 *Neisseria gonorrhoeae* clin.is. | **64** | **256** |
| 3292 *Moraxella catarrhalis* clin.is. | **16** | **64** |

The antibiotic is active *in vitro* against *Staphylococci* including Methicillin resistant *S. aureus* (MRSA), *Streptococci,* and *Enterococci.*

*S.aureus* can cause life-threatening infections and MRSA is of particular clinical significance because it is resistant to all penicillins and cephalosporins and also to multiple other antibiotics; in addition it easily spreads from patient to patient causing outbreaks of infection with important implications for healthcare facilities.

Vancomycin resistant *Enterococci* (VRE) are emerging as important hospital-acquired pathogens responsible for severe human infections (such as endocarditis, meningitis and septicemia) posing an increasing therapeutic challenge (Y. Cetinkaya, P. Falk and C.G. Mayhall. Vancomycin-resistant enterococci-Clin. Microbiol Rev.13 (2000) 686-707; L.B. Rice. Emergence of vancomycin-resistant enterococci. Emerg. Infec. Dis. 7 (2001) 183-7).

*S. pneumoniae* and *M. catarrhalis* are recognized important pathogens of humans. They are a common cause of respiratory tract infections, particularly otitis media in children and lower respiratory tract infections in the eldery. *M. catarrhalis* and *S. pneumoniae* have been recently accepted as the commonest pathogens of the respiratory tract (M.C. Enright and H. McKenzy. *Moraxella* (Branhamella) catarrhalis- Clinical and molecular aspect of a rediscovered pathogen. J. Med. Microbiol. 46 (1997) 360-71).

### IN VIVO BIOLOGICAL ACTIVITY OF ANTIBIOTIC 97518

Female ICR mice (Harlan, Italy) weighing 14-16 g were infected intraperitoneally (ip) with 6.5 x 10² cells of *Streptococcus pyogenes* C 203 in 0.5 mL of saline supplemented with 1% peptone. Antibiotic 97518 was administered intravenously (iv) or subcutaneously (sc) within 10-15 min after infection. The 50% effective doses (ED₅₀) were calculated by the Spearman-Kärber method (D.J. Finney. The Spearman-Kärber method. In: Statistical Methods in Biological Assay. (1952) pp. 524-530, Charles Griffin &Co., Ltd., London) from the percentage of animals surviving to day 7 at each dose.

Antibiotic 97518 gives ED₅₀ values of 3.75 mg/kg either when administered intravenously (iv) or subcutaneously (sc).

Antibiotic 97518 is not toxic up to 100 mg/Kg.

### MECHANISM OF ACTION OF ANTIBIOTIC 97518

*Staphylococcus aureus* strain ATCC6538P (L100) was grown overnight at 37 °C in a rotary bath in Iso-Sensitest Broth (Oxoid Ltd., Basingstoke, Hampshire, England). The culture was diluted up to an OD₅₄₀ = 0.03 in a 1:1 (v/v) mixture of Iso-Sensitest broth : Davis Mingioli Broth (Difco Labs, Detroit, USA) added with 1g/l of KH₂PO₄, and was incubated at 37°C. To determine the antibiotic dose/response effects on RNA, DNA, protein and cell wall biosyntheses, the culture was divided in four parts when the optical density reached 0.4. Individual precursors of DNA (2 Ci/l ³H-thymidine + 0.1g/l unlabelled adenosine), RNA (5 mCi/l ³H-uracil), protein (10 mCi/l ³H-leucine), and cell wall (1 mCi/l ³H-N-acetylglucosamine + 3.5 mg/l unlabelled N-acetylglucosamine) biosyntheses were then added, resptectively, to each one of the four aliquots and, immediately after the addition, 100 µl of the resulting culture was dispensed in wells of microtiter plate (96 wells format), containing 10 µl of solutions of antibiotic 97518. After 20 min of incubation at 37°C, 0.1 ml ice-cold 20% (w/v) trichloroacetic acid was added to stop the incorporation. After 30 minutes at 4°, the samples were filtered on Filtermat A (Wallac) using an 96 well Wallac Cell Harvester. The Radioactivity not incorporated was washed away sequentially with water and ethanol. The radioactivity of the biopolymers was counted on the filters, using a Beta Plate System (Wallac).

Antibiotic 97518 blocked cell wall synthesis completely at 2.5 µg/ml, whereas the other macromolecular syntheses were only partially inhibited at the same concentrations. This is indicative of a primary mode of action on cell wall synthesis. In control experiments, nisin inhibited all the four macromolecular syntheses at the same concentration.

### BRIEF DESCRIPTION OF THE DRAWINGS

FIG.1 represents mass spectra of antibiotic 97518 showing a doubly protonated ion at m/z 1097:
   A) Full-scan low resolution spectrum
   B) Zoom-scan high resolution spectrum
FIG.2 represents the I.R. absorption spectrum of antibiotic 97518 (in KBr).
FIG.3 represents the ¹H-NMR of antibiotic 97518, measured at 500 MHz in H₂0/D₂0/CD₃CN 80/10/10 (pH 2.6 HCl) at 25°C conc.5.9 mg/0.6 ml
FIG. 4 represents the ¹³C-NMR of antibiotic 97518, measured at 125 MHz in H₂0/D₂0/CD₃CN 80/10/10 (pH 2.6 HCl) at 25°C conc.5.9 mg/0.6 ml

The following examples further illustrate the invention and have not to be interpreted as limiting it in any way.

### Examples

### Example 1: Fermentation method for Planomonospora sp. DSM 14920

*Planomonospora* strain was maintained on oatmeal agar slants. The microbial content of one slant was scraped with 5 ml of sterile water and inoculated into three 500 ml Erlenmeyer flasks containing 100 ml of seed medium AF/MS which is composed of: (g/l) dextrose 20, yeast extract 2, soybean meal 8, NaCl l and calcium carbonate 4. Medium was prepared in distilled water and pH adjusted to 7.3 prior to sterilization at 121°C for 20 min.

The inoculated flasks were grown at 28°C, on a rotatory shaker operating at 200 rpm. After 5 days incubation, 5% of culture was inoculated into a second series of flasks containing the same fermentation medium. After 96 hours, 400 ml were transferred into 20 l Chemap bioreactors containing 15 l of the same flask medium. The fermenter was incubated for 68 hours at 28°C with 600 rpm stirring and 0.5 vvm aeration. This culture was then inoculated into production tank. The production of antibiotic 97518 was performed in two 300 l Bioengineering fermenters containing 200 l of the production medium M8 composed of (g/l): starch 20, glucose 10, yeast extract 2, hydrolyzed casein 4, meat extract 2 and calcium carbonate 3. The medium was prepared in deionised water at pH adjusted to 7.0 before sterilization at 121°C for 30 min. After cooling the fermenters were inoculated with about 14 l (7%) of pre-culture. Fermenters were run at 28°C, stirred at 180 rpm, aerated at 0.5 vvm. One of them was harvested after 96 hours of fermentation, whereas the second production fermenter was harvested after 260 hours. The production of the antibiotic 97518 was monitored by HPLC analysis, as described above in the Recovery and Purification Section.

### Example 2 Recovery and Purification of antibiotic 97518

The fermentation was harvested after 96 hours and the broth was filtered by tangential filtration (0,1 micron pore size membrane) to obtain 150 l of filtrate at pH 7,7. This solution was then stirred overnight at room temperature in the presence of 4 l of HP 20 polystyrene resin (Mitsubishi Chemical Co.). The resin was then recovered and eluted batch-wise with 20 l of methanol. The eluted solution, which contained antibiotic 97518, was concentrated under vacuum to an oily residue. This was suspended in 200 ml of distilled water and was lyophilized, yielding 102 g of crude 97518 preparation. Part of this material (89,6 g) was then suspended in 7 l of distilled water and was filtered to remove the insoluble residue. The filtrate was again adsorbed batch-wise on 2 l of HP-20 polystyrenic resin. The resin was filtered and was washed with 10 l of methanol: water 1:1 (v:v) mixture, which was discharged. Antibiotic 97518 was then eluted with 10 l of methanol:water 9:1 (v/v) mixture and then with 6 1 of methanol:n-buthanol:water mixture 90:5:10 (v/v/v) mixture. The eluates were pooled and were concentrated under vacuum to 70 ml. Upon addition of 700 ml of acetone a precipitate was obtained which was filtered, yielding 9,5 g of crude antibiotic 97518. Part of this preparation (3,6 g) was then processed by preparative HPLC. The purification was performed on a Lichrosorb RP8 (Merck, Darmstad) 25 x 250 mm column (7 µm particle size), which was eluted at 30 ml/min flow rate with mixtures of phase A (20 mM ammonium formate pH 4.5 buffer: CH₃CN 95:5) and phase B (20 mM ammonium formate pH 4.5 buffer:CH₃CN 5:95). The separation was performed by eluting according to the following sequence of linear gradients: a) from 10% to 25% of phase B in 10 min, b) from 25% to 30% of phase B in 32 min; c) from 30% to 90% of phase B in 5 min. Detection was at 230 nm.

Aliquots of 100 mg of crude antibiotic 97518, dissolved in 350 µl of 20 mM Tris buffer, pH 8.3, were processed per chromatographic run. The antibiotic typically eluted after 22 minutes. The eluted fractions of 35 repeated chromatographic runs, which contained antibiotic 97518, were then pooled, and were concentrated under vacuum. Water solutions of the resulting residue were then lyophilized three times, sequentially, yielding 320 mg of purified antibiotic 97518.

### Example 3: Alternative recovery and purification process of antibiotic 97518 preparation.

The fermentation was harvested after 260 hours, according to example 1, and the broth was brought to pH 3 with concentrated sulfuric acid. The broth was incubated overnight at 40° C and was then filtered under vacuum with Hyflo filter aid. The filtrate was then stirred overnight at room temperature in the presence of 41 of HP 20 polystyrene resin (Mitsubishi Chemical Co.). The resin was then recovered and washed batch-wise with 20 l of methanol: water 1:1 (v/v) mixture to remove impurities. Antibiotic 97518 was then eluted with 20 l of a methanol:n-butanol:water 9:1:1 (v/v/v) mixture. The eluate was concentrated under vacuum to 11 residue from which 40 g of crude antibiotic 97518 precipitated upon addition of 3 l of acetone. Part of this material (33.6 g) was then suspended in 11 of methanol:water 1:1 (v/v) and was stirred for 15 min at 55° C. The suspension was then cooled overnight at 4°C and the solid was recovered by centrifugation. This solid was again suspended in 0.5 l of methanol: water 1:1 (v/v) mixture, which was processed as above described. The liquid phases were discharged and the solid residue was collected. This material was dissolved in distilled water and was lyophilized, yielding 21.3 g of crude antibiotic 97518.

Part of this antibiotic 97518 preparation was processed by preparative HPLC. The purification was performed on a Lichrosorb RP8 (Merck, Darmstad) 25 x 250 mm column (7 µm particle size), which was eluted at 30 ml/min flow rate with mixtures of phase A (20 mM ammonium formate pH 4.5 buffer) and phase B (CH3CN). The separation was performed by eluting according to the following sequence of linear gradients: a) from 10% to 25% of phase B in 10 min, b) from 25% to 40% of phase B in 32 min; c) from 30% to 90% of phase B in 5 min. Detection was at 230 nm. The crude antibiotic (600 mg) was dissolved in 2 ml of DMSO and 300 µl of this solution were processed per chromatographic run. Antibiotic 97518 typically eluted after 26 min. The eluted fractions of repeated chromatographic runs, which contained antibiotic 97518, were then pooled, were concentrated under vacuum and were then lyophilized. The solid was re dissolved in water and was again lyophilized, yielding 255 mg of purified antibiotic 97518.

## Claims

1. Antibiotic 97518 having the following characteristics:
A) Mass spectrum (Fig. 1A) recorded from a 0.01 mg/ml solution in acetonitrile: water 50:50 (v/v) giving a doubly protonated ion at 1097 m/z on a Thermofinnigan LCQ deca instrument under the following electrospray conditions: spray voltage: 4.5 kV; capillary temperature: 200°C; capillary voltage: 28 V; infusion mode 5µl/min;
B) Infrared spectrum (Fig. 2), recorded in KBr with a Bruker FT-IR spectrophotometer model IFS 48, exhibiting the following absorption maxima (cm⁻¹): 3295; 3058; 2970; 2930; 1660; 1531; 1411; 1336; 1263; 1109;
C) The U.V. spectrum, performed in MeOH with a Perkin-Elmer spectrophotometer Lambda 16, exhibiting two shoulders at 279 and 288 nm;
D) ¹H-NMR spectrum (Fig. 3) recorded in the mixture H₂O/D₂O/CD₃CN 80/10/10 (pH 2.6 HCl) at 25°C on a Bruker AMX 600 spectrometer applying a water suppression sequence, using as internal standard the residual signal of acetonitrile at 1.99 ppm, showing the following ¹H signals:
[δ=ppm multiplicity; (attribution)]: 0.79 d (CH₃), 0.84 d (CH₃), 0.86 d (CH₃), 0.97 d (CH₃), 1.1 d (CH₃), 1.20 d (CH₃), 1. 71 d (CH₃), 2.33 q (CH₂), 2.4 - 3.6 m (peptidic alpha CH' s), 3.6 - 5.1 m (peptidic alpha CH's), 5.27 - 5.82 s (CH₂), 6.49 q (CH), 6.9 - 8.57 t and d (aromatic CH's), 7.74 - 9.1 d and m (aromatic CH's and peptidic NH's);
E) ¹³C-NMR spectrum (Fig. 4) recorded in the mixture H₂O/D₂O/CD₃CN 80/10/10 (pH 2.6 HCl) at 25°C on a Bruker AMX 600 spectrometer using as internal standard the residual signal of acetonitrile at 1.39, 118.69 ppm showing the following ¹³C signals: [δ=ppm; (attribution)]: 10.27 - 20.95 (aliphatic CH₃' s) , 23.42 - 48.15 (aliphatic CH₂'s), 48.77 - 66.6 (peptidic alpha CH's), 108 - 136 (aromatic and double bonds CH's and quaternary carbons), 166.12 - 176.3 (peptidic carbonyls);
F) The acid hydrolysate (HCl 6N, 105°C, 24 h) showing the presence of the following acid resistant amino acids after derivatization with 6-aminoquinolyl-N-hydroxysuccinimidyl carbamate: lanthionine, methyllanthionine, glycine, threonine, proline, valine, glutamic acid, histidine and isoleucine; and its salts with acids and bases.

2. A process for producing antibiotic 95518 and its salts with acids and bases as defined in claim 1, which comprises:
- cultivating Planomonospora sp. DSM 14920, or a variant or mutant thereof maintaining the ability of producing said antibiotic, under aerobic conditions, in an aqueous nutrient medium containing an assimilable source of carbon, nitrogen and inorganic salts;
- isolating the resulting antibiotic from the mycelium and/or the filtered culture broth;
- purifying the isolated antibiotic.

3. A process according to claim 2, wherein the Planomonospora sp. DSM 14920 or the antibiotic 97518 producing variant or mutant thereof are pre-cultured.

4. A process according to any of claims 2 and 3 wherein the isolation of the antibiotic is carried out by filtering the culture broth at a pH between 7 and 8, contacting the filtered broth with an absorption matrix, preferably a polystyrene or mixed polystyrene-divinylbenzene resin, and eluting said resin with a polar, water miscible solvent or a mixture thereof, preferably methanol, whereby a solution containing the crude antibiotic is obtained.

5. A process as in claim 4 wherein the culture is brought to pH 3 before filtration and the resin with which the filtered broth is contacted is washed with a mixture of water and methanol and then eluted with a mixture of methanol/n.butanol and water whereby a solution containing the crude antibiotic is obtained.

6. A process according to any of claims 2 to 5 wherein the isolation of the antibiotic comprises the extraction from the mycelium with methanol, whereby a solution containing the crude antibiotic is obtained.

7. A process according to any of claims 2 and 3 wherein the isolation of the antibiotic is carried out by extracting the fermentation broth with a water immiscible solvent whereby a solution containing the crude antibiotic is obtained.

8. A process as in any of claim 4 to 7 whereby the crude antibiotic is recovered from the solution containing it by concentration or by addition of a non-solvent.

9. A process as in any of claims 2 to 8 wherein the antibiotic is purified by means of preparative chromatography.

10. A pharmaceutical composition comprising the antibiotic according to claim 1 or its pharmaceutically acceptable salts with acids or bases.

11. A pharmaceutical composition according to claim 10, comprising a pharmaceutically acceptable carrier.

12. The antibiotic according to claim 1 and its pharmaceutically acceptable salts with acids or bases for use as a medicament.

13. Use of the antibiotic according to claim 1 and its pharmaceutically acceptable salts with acids or bases for the manufacture of a medicament for the treatment of bacterial infections.

14. Use of the antibiotic according to claim 1 and its non-toxic salts as animal growth promoter.

15. A biologically pure culture of the strain *Planomonospora* Sp DSM 14920, or a variant or mutant thereof maintaining the ability of producing the antibiotic 97518 of claim 1 when cultivated under submerged aerobic conditions in the presence of assimilable sources of carbon, nitrogen and inorganic salts.
